# EUROPEAN PATENT SPECIFICATION

(11) **EP 4 097 080 B1**
(45) Date of publication and mention of the grant of the patent: **22.04.2026**
(21) Application number: 21747334.7
(22) Date of filing: 27.01.2021
(51) Int. Cl.: C07C 315/02, B01D 3/00, B01D 5/00, C07C 317/04, C07C 319/14, C07C 321/14, D21C 11/04, C07C 29/74, C07C 31/04, C07C 45/78, C07C 315/06, C07C 319/26, C07C 29/00, C07C 29/80, D21C 11/00

(54) **METHOD AND PROCESS PLANT FOR TREATMENT OF A STREAM OF MIXED COMPOUNDS**
VERFAHREN UND VERFAHRENSANLAGE ZUR BEHANDLUNG EINES STROMES GEMISCHTER VERBINDUNGEN
PROCÉDÉ ET INSTALLATION DE TRAITEMENT POUR LE TRAITEMENT D'UN FLUX DE COMPOSÉS MIXTES

(30) Priority: 28.01.2020 SE 2050074
(43) Date of publication of application: 07.12.2022
(73) Proprietor: Södra Skogsägarna Ekonomiska Förening, 351 89 Växjö (SE)
(72) Inventor: PARKÅS, Jim, 432 94 Varberg (SE); ISAKSSON, Johan, 42834 Kållered (SE); SOLHAGE, Fredrik, 504 34 Borås (SE)
(74) Representative: AWA Sweden AB
(86) International application number: PCT/SE2021/050047
(87) International publication number: WO 2021/154143

(56) References cited:
- EP-B1- 2 670 908
- WO-A1-2015/053704
- SE-C2- 539 579
- US-A- 2 816 832
- US-A- 2 976 273
- US-A- 5 718 810
- VON SCHENCK, ANNA; JANSSON, MIKAEL; LJUNGQUIST, PIERRE: "Rening av metanol för användning som fordonsbränsle = Cleaning of methanol for transportation use", RAPPORT, vol. Projekt S 5-628, 30 November 2007 (2007-11-30), pages 1 - 29, XP009538951, ISSN: 1653-1248

## Description

### TECHNICAL FIELD

This invention relates to a method and use of a process plant for treatment of a stream of mixed compounds obtained from a process comprising decomposition and/or conversion of a wood or pulp material.

### BACKGROUND OF THE INVENTION

There is an increasing interest in bio-based chemicals, fuels and materials and thereby also an interest in increasing efficiency and flexibility of methods and process plants using bio-material as raw material. An example in this field of technology is disclosed in SE539579C2 where raw methanol obtained as a by-product in the kraft or sulphate process of pulp production in a pulp mill is purified by removing e.g. sulphurous compounds. The purified methanol may then be used as a non-fossil fuel or as a component in other processes.

WO 2015/053704 describes a method for purifying raw methanol containing sulphurous compounds, wherein the method comprises a step of washing raw methanol with a non-polar organic solvent. The non-polar organic solvent is white mineral oil, white oil, mineral oil, paraffin oil, C8-C20 alkanes or triglycerides (which are liquid at temperature below 40 °C).

Von Schneck et al., ISSN 1653-1248, describes a techno-economic analysis on the possibility of separating malodours sulphur compounds from the methanol obtained in the condensate after the evaporation in the kraft pulping process.

US 5 718 810 describes a method for recovering methanol from sulphur-based wood pulping process vapours, which contain at least methanol and dimethyl disulfide (DMDS). The method involves three steps. The first step consists of introducing the vapours into a first distillation tower at a vapor introduction point. The second step consists of introducing water into the first tower at a point above the vapor introduction point. The third step consists of heating liquid which is in the first tower below the vapor introduction point so that a liquid stream flows from the bottom of the first tower and a gas stream flows from the top of the first tower. The liquid stream contains most of the methanol originally in the vapours. The gas stream contains most of the dimethyl disulfide originally in the vapours. The liquid stream can then be distilled in a second distillation tower by conventional distillation to produce dry methanol. The first and third steps can be combined by introducing sufficiently heated vapours. When the vapours contain more than sixty percent water, then there need be only two steps. The first step consists of introducing the vapours into a first distillation tower at a vapor introduction point. The second step consists of heating liquid which is in the first tower below the vapor introduction point so that a liquid stream flows from the bottom of the first tower and a gas stream flows from the top of the first tower.

US 2,816,832 discloses a process for making dimethyl sulphide from pulp mill spent liquor which may be applied without destroying the material balance employed in the pulping procedure.

US 2,976,273 shows that considerable quantities of dimethyl sulphide can be produced from kraft black liquor which does not contain any sulphide or bisulfide but in which the sulfidic sulphur has been oxidized for example prior to concentrating to avoid sulphur losses in that operation.

### SUMMARY OF THE INVENTION

An object of this invention is to provide an improved method and use of a process plant for handling and utilizing a stream of bio-based raw material, such as the raw methanol used as raw material in SE539579C2. This object is achieved by the method and use of a process plant defined by the technical features contained in the corresponding independent claims. The dependent claims contain advantageous embodiments, further developments and variants of the invention.

The invention concerns a method for treatment of a stream of mixed compounds obtained from a process comprising decomposition and/or conversion of a wood or pulp material, the method comprising: feeding the stream through a processing arrangement comprising one or more treatment units arranged to separate methanol from other compounds in the stream and form a first product flow containing methanol, wherein the one or more treatment units comprises at least a first primary separation unit arranged to separate one or more compounds other than methanol from the stream.

The method further comprising: feeding the compounds separated from methanol in the first primary separation unit to a first auxiliary separation unit so as to separate a second compound from at least one of the other compounds separated from methanol in the first primary separation unit and thereby increase the purity of said second compound and form a second product flow in the form of a purified second compound and/or a purified derivative of the second compound, wherein the second compound is dimethyl sulphide (DMS, CH3-S-CH3), methyl mercaptan (CH3-S-H) or acetone (CH3-CO-CH3).

The method of this disclosure thus produces other compounds, such as DMS, DMSO (dimethyl sulfoxide, oxidized derivative of DMS) and/or acetone, as a complement or alternative to methanol. This allows for a selection of which product compound(s) to focus on when operating the method, i.e. the method allows for optimizing production (or purity) with regard to the selected product compound(s). As a result, the method becomes flexible and efficient. For example, if the demand for purified DMS increases, the method can be operated to increase production of DMS while decreasing production of methanol. As will be further described below, the method of this disclosure also provides for increased flexibility and efficiency by the use of additional compound flows within the process arrangement or flows fed into the processing arrangement from an adjacent paper mill. For instance, sulphur compounds (or *sulphur* compounds, both spellings are used in this disclosure) separated in the processing arrangement, such as H₂S, may be used to convert methanol to DMS in a recirculation arrangement or produce additional DMS by reaction with lignin obtained from black liquor treatment of wood material.

The method of the present disclosure is in contrast to e.g. SE539579 where the focus is set only on purifying methanol and where no particular attention is paid to several of the compounds removed from the raw methanol, such as the volatile compounds and acetone. As explained in more detail below, the present method may produce purified methanol as one of the purified products but may alternatively be operated without any purpose of purifying methanol and instead even use methanol as a raw material for producing other compounds. The first product flow containing methanol has in such a case a relatively low mass flow rate and a low purity of the methanol. Such a product flow may be used for combustion and providing heat to e.g. the processing arrangement.

In the method of this disclosure, methanol may be stepwise purified when the raw material stream is fed through the processing arrangement (which may be denoted "biorefinery"). Similar treatment units as used in SE539579, such as the distillation units, may be used also in the method described here. However, the type(s) and number of treatment units required for the present method depends on the particular application.

The first primary separation unit defined above (which is named "first" just to specify a separation unit that forms part of the processing arrangement, not necessarily because it has a certain position in a series of separation units) is typically a distillation unit or stripper but may instead be configured for membrane-filtering or other separation. The separation unit handling the raw material stream is here denoted primary separation unit to distinguish from the auxiliary separation unit. The second compound separated in the first auxiliary separation unit may be a volatile evaporated fraction or a liquid fraction from a condenser-type of auxiliary separation unit. As will be described further below, the process arrangement preferably comprises also a second primary separation unit and a corresponding second auxiliary separation unit so as to allow separation of a third purified compound, such as acetone, in addition to the second compound, such as DMS, that in such a case may be separated and purified at the first (primary) separation unit upstream of the second (primary) separation unit. The auxiliary separation units do not handle the raw material stream but only the compounds separated from that stream in the corresponding primary separation unit.

As to the term "purified", e.g. "purified second compound", this generally means sufficiently purified for the intended purpose. As is well known in the field, a chemical compound is never 100% pure and increasing purity is almost always connected to an increasing cost. If "purified methanol" is to be produced, e.g. as a purified methanol in the first product flow, the purity may be >90, >95, >99, >99.5, >99.85 or >99.9 weight %. For DMS, methyl mercaptan and acetone, the purity of the "purified compound" typically may be >90, >95. >99 or >99.9 weight %. The process arrangement can be adapted to reach certain degrees of product purity, for instance by including additional auxiliary separation units and/or recirculating selected flows. The process arrangement may also be operated in different ways to reach certain degrees of product purity, the temperature in a certain separation unit may for instance be increased or decreased.

The term "product flow" indicates an outgoing flow from a treatment unit and thus a flow of some kind of product formed in the process. But it is not necessarily so that a product flow, in particular not the first product flow, contains only one purified compound product that is taken out from the processing arrangement or the process plant where the processing arrangement is located. A product flow may be used as an incoming flow at some other point in the process plant. Purity of methanol may be low in the first product flow, in particular if it is used in the process as a reactant for producing additional amounts of DMS, and if so the first product flow may be used as a fuel product.

In an embodiment the one or more treatment units further comprises a second primary separation unit arranged to separate methanol from other compounds in the stream, wherein the method comprises: feeding the compounds separated from methanol in the second primary separation unit to a second auxiliary separation unit so as to separate a third compound from at least one of the other compounds separated from methanol in the second primary separation unit and thereby increase the purity of said third compound and form a third product flow in the form of a purified third compound or a purified derivative of the third compound, wherein the third compound is dimethyl sulphide (DMS, CH3-S-CH3), methyl mercaptan (CH3-S-H) or acetone (CH3-CO-CH3), and wherein the second and third compounds are different compounds.

In embodiments the first and/or the primary separation unit is a distillation unit.

In an embodiment the processing arrangement comprising at least two treatment units arranged in series in relation to the flow of the stream containing methanol.

In an embodiment the processing arrangement is arranged to be capable of purifying methanol until it forms a first product flow in the form of a purified methanol.

In an embodiment the first primary separation unit is arranged to separate compounds from the stream that have a boiling point that is lower than that of methanol.

In an embodiment the first auxiliary separation unit is based on deviating boiling points.

In an embodiment the second primary separation unit is arranged to separate compounds from the stream that have a boiling point that is lower than that of methanol.

In an embodiment separation in the second auxiliary separation unit is based on deviating boiling points.

The first product flow may contain methanol, ethanol and water. Further separation units may be used to increase the purity of methanol and/or ethanol if desired.

In an embodiment the second compound is dimethyl sulphide (DMS, CH3-S-CH3) or methyl mercaptan (CH3-S-H). DMS may be separated from e.g. methyl mercaptan in the first auxiliary separation unit. Another compound that may be separated from DMS in the first auxiliary separation unit is H₂S. As an example, DMS is the second compound and purified DMS (or DMSO) forms the second product flow, whereas methyl mercaptan (and other compounds separated from DMS) forms a flow used at another location of the process arrangement. In another example, also purified methyl mercaptan forms a product flow, whereas H₂S, purified or in a mix with some other compounds, forms a (product) flow used at another location of the process arrangement.

In an embodiment the third compound is acetone (CH3-CO-CH3).

In an embodiment where the second compound is dimethyl sulphide (DMS, CH3-S-CH3) or methyl mercaptan (CH3-S-H), the third compound is acetone (CH3-CO-CH3). Typical acetone impurities, such as methanol and volatile sulphur compounds, may be separated and removed from acetone in the second auxiliary separation unit. An additional auxiliary separation unit may be included to, for instance, remove methanol from acetone in the second auxiliary separation unit and remove more volatile impurities in the additional auxiliary separation unit.

In an embodiment the first primary separation unit is arranged upstream of the second primary separation unit in relation to the flow of the stream containing methanol.

In an embodiment the method comprises: operating the processing arrangement so as to increase a mass flow rate of the second compound in the second product flow while decreasing a mass flow rate of methanol in the first product flow.

This can be done by e.g. changing temperature or pressure in the first primary separation unit so that a larger fraction of the second compound is separated from the stream containing methanol. This generally also results in that a larger fraction of methanol follows the second compound in the separation. This accompanying methanol can be removed in an auxiliary separation unit if desired to increase purity of the second compound. In effect this typically leads to a reduced total amount of methanol in the outgoing stream from the first primary separation unit and thereby to a reduced amount/concentration of methanol in the first product flow. Similar can be said for the third compound and the second primary separation unit. Some of methanol, such as methanol, can thus be spent on producing more of the second product, such as DMS or acetone, when so desired.

Another way of allowing increased production of the second (or third) compound is to recirculate a portion of the stream containing methanol so as to pass it through a primary separation unit an additional time.

In an embodiment the method comprises: recirculating a portion of the stream in a recirculation flow line from a point in the processing arrangement downstream of a separation unit where DMS and/or methyl mercaptan is separated from the stream, to a point in the processing arrangement upstream of the separation unit where DMS and/or methyl mercaptan is separated from the stream, wherein the recirculated portion of the stream during recirculation is fed through a DMS/methyl mercaptan production reactor to which is fed, via a supply line, one or more sulphur compounds capable of reacting with methanol or another compound in stream so as to form DMS and/or methyl mercaptan.

In such an embodiment the production of DMS/methyl mercaptan can be increased by increasing the portion of the stream that is recirculated. The downstream point where the recirculation of the stream starts can in principle be anywhere downstream the separation unit in question, i.e. directly downstream that separation unit or downstream one or more further treatment units. As methanol is converted to DMS or methyl mercaptan in the production reactor, the production of methanol will at the same time decrease. The composition of the sulphur compounds and the mass flow rate of the sulphur compounds fed to the production reactor may be varied depending on desired outcome of products from the process arrangement. Relative production of DMS versus methyl mercaptan depends e.g. on the amounts of H₂S fed to the reactor. The sulphur compounds used for this reaction may include H₂S, and these sulphur compounds may be compounds that were present in the incoming raw material stream and now can be obtained from one or more of the treatment units of the process arrangement. Alternatively, or as a complement, these sulphur compounds may be supplied from outside of the process arrangement (e.g. from some other part of an integrated plant).

In an embodiment wherein the second compound is DMS, the method comprises: feeding purified DMS from the first auxiliary separation unit to an oxidizing unit so as to oxidize DMS and form a purified derivative of DMS in the form of dimethyl sulfoxide (DMSO).

In an embodiment the method comprises: feeding a flow containing DMS and/or methyl mercaptan from a lignin treatment arrangement to a point in the processing arrangement upstream of a separation unit where DMS and/or methyl mercaptan is separated from the stream, wherein the lignin treatment arrangement is arranged to react at least a portion of a flow of incoming lignin obtained from a wood or pulp decomposition and/or conversion process with sulphur from an incoming flow of sulphur compounds so as to form DMS and/or methyl mercaptan.

This is a particularly suitable embodiment when the plant is an integrated plant comprising both the processing arrangement, i.e. the bio-refinery, as well as a pulp production process including a "wood or pulp decomposition and/or conversion process" where wood material typically is digested in black liquor. This forms an additional way of increasing the production of DMS (as well as reactive lignin formed in the lignin treatment arrangement).

In an embodiment the stream of mixed compounds is obtained from black liquor used for digestion of wood material in pulp production in a pulp mill. In particular, the stream of mixed compounds may be a condensate from evaporation of such black liquor. Alternatively, the stream of mixed compounds may be obtained from some other decomposition of wood material in e.g. a bio-refinery.

The invention also concerns a use of a process plant for treatment of a stream of mixed compounds obtained from a process comprising decomposition and/or conversion of a wood or pulp material, the process plant comprising: a processing arrangement comprising one or more treatment units configured to, when the stream is fed through the processing arrangement, separate at least methanol from other compounds in the stream and form a first product flow containing methanol, wherein the one or more treatment units comprises at least a first primary separation unit arranged to separate one or more compounds other than methanol from the stream; the processing arrangement further comprising a first auxiliary separation unit arranged in association with the first primary separation unit so as to receive the compounds separated from methanol in the first primary separation unit, the first auxiliary separation unit being configured to separate a second compound from at least one of the other compounds separated from methanol in the first primary separation unit and thereby increase the purity of said second compound and form a second product flow in the form of a purified second compound and/or a purified derivative of the second compound.

**In** an embodiment of the use of a process plant, the one or more treatment units of the process plant further comprises a second primary separation unit arranged to separate methanol from other compounds in the stream, and wherein the processing arrangement further comprising a second auxiliary separation unit arranged in association with the second primary separation unit so as to receive the compounds separated from methanol in the second primary separation unit, the second auxiliary separation unit being configured to separate a third compound from at least one of the other compounds separated from methanol in the second primary separation unit and thereby increase the purity of said third compound and form a third product flow in the form of a purified third compound and/or a purified derivative of the third compound.

In an embodiment of the use of a process plant, the process plant comprises a process configured to decompose and/or convert a wood or pulp material and form the stream of mixed compounds. In a variant the process is configured for black liquor digestion of wood material and wherein the stream of mixed compounds is obtained from the black liquor.

In an embodiment of the use of a process plant, the processing arrangement comprises at least two treatment units arranged in series in relation to the flow of the stream containing methanol.

In an embodiment of the use of a process plant, the processing arrangement is arranged to be capable of purifying methanol until it forms a first product flow in the form of a purified methanol.

In an embodiment of the use of a process plant, the first primary separation unit is arranged to separate compounds from the stream that have a boiling point that is lower than that of methanol.

In an embodiment of the use of a process plant, the first auxiliary separation unit is arranged to separate compounds based on deviating boiling points.

In an embodiment of the use of a process plant, the second primary separation unit is arranged to separate compounds from the stream that have a boiling point that is lower than that of methanol.

In an embodiment of the use of a process plant, the second auxiliary separation unit is arranged to separate compounds based on deviating boiling points.

In an embodiment of the use of a process plant, the first primary separation unit is arranged upstream of the second primary separation unit.

In an embodiment of the use of a process plant, the processing arrangement is configured to be operated so as to increase a mass flow rate of the second product flow while decreasing a mass flow rate of the first product flow.

In an embodiment of the use of a process plant, the processing arrangement comprises: a recirculation flow line arranged to recirculate a portion of the stream from a point in the processing arrangement downstream of a primary separation unit where DMS and/or methyl mercaptan is separated from the stream, to a point in the processing arrangement upstream of the primary separation unit where DMS and/or methyl mercaptan is separated from the stream; a DMS/methyl mercaptan production reactor through which the portion of the stream is fed during recirculation; and a supply line for feeding one or more sulphur compounds to the DMS/methyl mercaptan production reactor, wherein the sulphur compounds are capable of reacting with methanol or another compound in stream so as to form DMS and/or methyl mercaptan.

In an embodiment of the use of a process plant, the processing arrangement comprises an oxidizing unit arranged downstream the first auxiliary separation unit so as to allow oxidation of the second compound and form a purified derivative of the second compound.

In an embodiment of the use of a process plant, the process plant comprises a lignin treatment arrangement arranged to react at least a portion of a flow of incoming lignin obtained from a wood or pulp decomposition and/or conversion process with sulphur from an incoming flow of sulphur compounds so as to form DMS and/or methyl mercaptan, wherein the use of a process plant further comprises a flow line for feeding DMS and/or methyl mercaptan from the lignin treatment arrangement to a point in the processing arrangement upstream of a separation unit where DMS and/or methyl mercaptan is separated from the stream.

In an embodiment of the use of a process plant, the second compound is dimethyl sulphide (DMS, CH3-S-CH3), methyl mercaptan (CH3-S-H) or acetone (CH3-CO-CH3).

In an embodiment of the use of a process plant, the third compound is dimethyl sulphide (DMS, CH3-S-CH3), methyl mercaptan (CH3-S-H) or acetone (CH3-CO-CH3), and wherein the second and third compounds are different compounds.

In an embodiment of the use of a process plant, the second compound is dimethyl sulphide (DMS, CH3-S-CH3) or methyl mercaptan (CH3-S-H) and the third compound is acetone (CH3-CO-CH3).

Aspects of the method of this disclosure may also be described according to the following points:
The raw material composition, e.g. a raw methanol composition, a black liquor being treated with an alkaline solution and/or a sulphur source, or an acetone rich material obtained from a partial or a total condenser e.g. an acetone column total condenser, comprises a number of chemical components, wherein each of said chemical components can be separated by the process according to the present invention, as described herein, by comprising said treatment step, e.g. comprising different treatment steps, and made into products, e.g. desirable products. The same products can, for example, be produced by utilizing raw methanol composition, black liquor or lignin as the raw material composition.

The raw material composition is a stream of mixed compounds obtained from a process comprising decomposition and/or conversion of a wood or pulp material.

The process enables production of one, or more, compounds according to formula (I) within a, so called, biorefinery concept, wherein the process provides further developments of the biorefinery concept, for example, regarding production of a multiple products portfolio from raw methanol, lignin and black liquor.

With a biorefinery concept, the production is maximized on the output from both a technical, sustainability and economical perspective. This results in an increased valorisation, by producing a multiple product portfolio for e.g. the chemical or energy sector, rather than maximizing the volume of only one product type.

The process has currently been constructed, and in the process, impurities may be removed from methanol containing stream to obtain marketable commodities by meeting international standards. This may be done through several separation steps known as such (see e.g. SE539579C2) including acidification, distillation and extraction. During one of these stages, lights may be distilled off, containing mainly hydrogen sulphide (H₂S), methyl mercaptan and dimethyl sulphide (DMS) (for example, around 30% DMS). The gaseous stream can be sent to combustion for energy recovery, however, e.g. DMS has a value as a commodity and could be separated from the mixture by differences in boiling points. Apart from the initial thermal separation of, e.g. DMS, optionally, some final polishing, e.g. process steps, may be performed to reach further improved purity, including an acceptable purity. In some instances, the process, in accordance with the present invention, produces, e.g. DMS, for example, 90 weight %, or more of DMS, 95 weight %, or more of DMS, or, alternatively, 99 weight %, or more of DMS.

Another aspect is to utilize as much of a raw material as possible, by numerous processes that combined results in a maximized resource efficiency. This can be part of a sustainability strategy since it maximizes the amount of products and minimizes the amount of low value by-products or even waste.

In embodiments of the process said raw material composition comprises, or is, a raw methanol composition and said process comprises addition of sulfuric acid, prior to said retrieval of said retrieving composition. Further, when the raw material composition comprises, or is, a raw methanol composition, the material composition consists of a number of chemical components, wherein each of said chemical components can be separated by the process by comprising said treatment step, e.g. comprising different treatment steps, and made into products, e.g. desirable products.

In further embodiments of the process said raw material composition comprises, or is, a black liquor being treated with an alkaline solution and/or a sulphur source.

In still further embodiments of the process, lignin in alkali, e.g. black liquor, may be treated with sulphur, or other sources of sulphur such as hydrogen sulphide, and methyl mercaptan, e.g. at 200-250°C, which may also render a lignin product with improved reactivity towards formaldehyde (demethylated lignin), i.e. a reactive lignin product. The demethylation of lignin gives an increased content of catechol structures in the lignin polymer which are known to exhibit higher reactivity, for instance, in reactions with formaldehyde. Said lignin product, i.e. the reactive lignin, can be isolated by any of the methods found in the literature, e.g. precipitation by adding acid which may also be combined with membrane filtration.

In further embodiments of the process said material composition and/or said raw material composition further comprises an internal source of sulphur. The sulphur source may be external such as purchased sulphur source but may also be internal. The internal source of nucleophilic sulphur compounds may be strong gases from the pulping process or from the biorefinery process itself where a source of reduced sulphur is available. The sulphur gases in kraft pulp mills are primarily burnt in order to minimize Odor problems (destruction).

In still further embodiments of the process said raw material composition is a black liquor being treated with an alkaline solution.

In even further embodiments of the process, said material composition and/or said raw material composition further comprises an internal source of sulphur, wherein the internal source of sulphur may comprise H₂S and CH₃SH from a methanol source, e.g. a methanol factory and, optionally, also strong gases from a Kraft pulp mill scrubbed in alkali giving HS⁻ and CH₃S⁻.

Further, in said oxidation step, DMS may be oxidized by using an oxidizing agent to DMSO, for example, by using oxygen as an oxidizing agent, e.g. by using a gas mainly composed of oxygen an oxidizing agent, and using a catalyst, e.g., using NOx as a catalyst in a liquid phase, wherein NOx may be any one or more of NO, N₂O₃, NO₂ and N₂O₄, for example a mixture of NO, N₂O₃, NO₂ and N₂O₄. In a further example of said oxidation step, DMS may be oxidized with nitrogen dioxide in nitrogen gas to DMSO, and nitrogen oxide nitrogen oxide may be formed which may be reacted back to NO₂ with addition of ammonia. Thus, when the compound of formula (**I**) is DMSO, said treatment step comprises an oxidation step wherein DMS has been oxidized.

### BRIEF DESCRIPTION OF DRAWINGS

In the description of the invention given below reference is made to the following figure, in which:
Fig. 1 schematically shows a process for producing "Product 1", "Product 2", "Product 3" and/or "Product 4" from a raw material stream.
Fig. 2 schematically shows a process for producing DMS and/or DMSO, and acetone from a raw material stream in the form of crude methanol.
Fig. 3 schematically shows a process for producing additional amounts of DMS and/or DMSO from a raw material stream in the form of crude methanol, wherein the process also comprises DMS produced from lignin.
Fig. 4 schematically shows a process for producing additional amounts of DMS and/or DMSO from a raw material stream in the form of crude methanol, wherein also some methanol is converted to DMS.
Fig. 5 schematically shows a process for producing DMS and/or DMSO from a raw material stream in the form of contaminated methanol.
Fig. 6 schematically shows an example of a use of a process plant according to this disclosure.

### DESCRIPTION OF EXAMPLE EMBODIMENTS OF THE INVENTION

### Example 1:

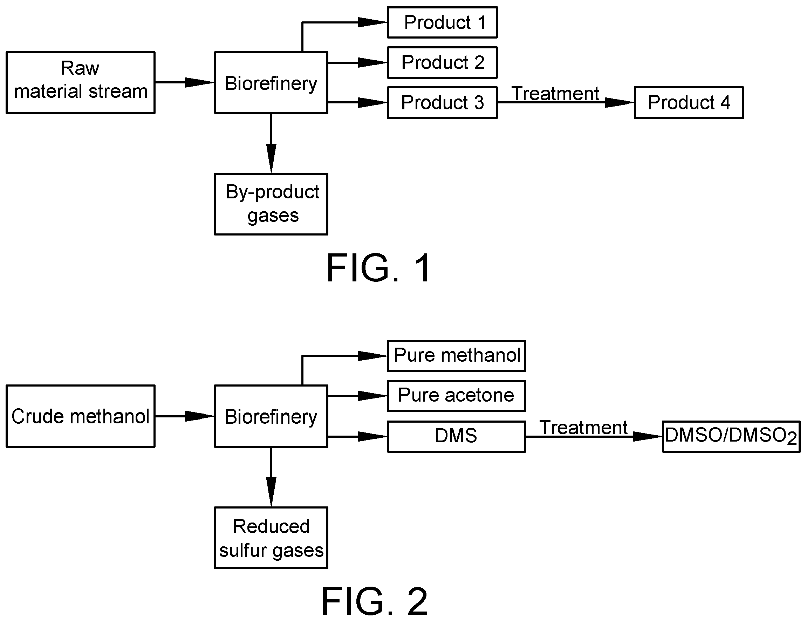

With reference to Fig. 1, a process within the biorefinery concept (see e.g. "Biorefinery" in Fig. 1), in accordance with the present invention, is described. Thus, in Fig. 1, a process, within the biorefinery concept, for producing "Product 1" (e.g. methanol, for example, pure methanol), "Product 2" (e.g. acetone, for example, acetone), "Product 3" (for example, DMS) and/or "Product 4" (for example, DMSO and/or DMSO₂), i.e. a process for producing one, or more, compounds according to formula (**I**). In some embodiments, "Product 1", "Product 2", "Product 3", and/or "Product 4" (see Fig. 1) are formed and being comprised in said material composition of said process. In embodiments, "Product 1", "Product 2", and/or "Product 3" (see Fig. 1) may be one, or more, compounds of formula (**II**) and may be comprised in said retrieving composition of said process. Further, said retrieving composition, comprising one, or more, of "Product 1", "Product 2", and "Product 3" (see Fig. 1) may be retrieved from "Raw material stream" (see Fig. 1), i.e. from said raw material composition. Furthermore, "By-product gases" (see Fig. 1), e.g. reduced sulphur gases, may be separated in the process. Further, with reference to Fig. 1, a process is described wherein said process comprises a selection of said treatment step, wherein the treatment step comprises one, or more, of a reaction step, a synthezising step, an oxidation step, a separation step, a stripping step, and a distillation step. Said selected treatment step may, e.g., be performed at, for example, "Biorefinery" in Fig. 1 and/or "Treatment" in Fig. 1.

### Example 2:

DMS can be produced starting from a raw material composition in accordance with the present process. The process here in Example 2 may further comprise treatment of lignin in black liquor with sulphur, or with other sources of sulphur such as hydrogen sulphide, and methyl mercaptan, e.g. at 200-250°C, i.e. treatment steps of the process.

### Example 3:

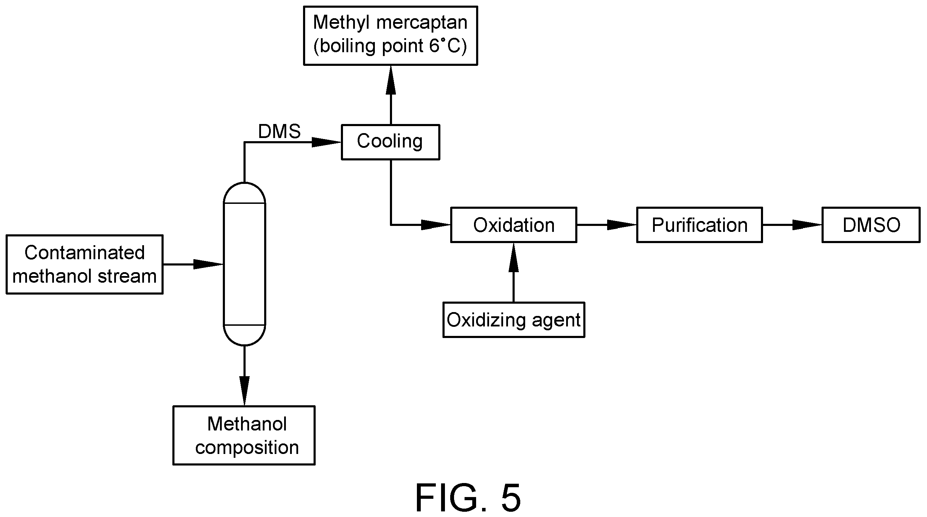

From a stripper top (for example, "Distillation" with "Volatiles"-stream in figure 1 of SE 539 579 C2) a DMS rich stream gas phase, was characterized to contain see table 1, where (MM) is methyl mercaptan, where H2S is hydrogen sulphide, where (DMDS) is dimethyl disulfide and (%) is weight %. Here in Example 3, a process comprising a DMS rich stream gas phase from such a stripper top, is described. Thus, Example 3 refers to the stripper top in Fig.5, i.e. the stripper top where "contaminated methanol stream", i.e. the raw material composition, is going in from the left and "methanol composition" is going out at the bottom, and "DMS", i.e. a retrieving composition, or a material composition, is going out in the top.

**Table 1**

| Sample | MM (%) | H2S (%) | DMS (%) | DMDS (%) |
|---|---|---|---|---|
| 1 | 5,82 | 2,45 | 2,33 | 0,01 |
| 2 | 6,22 | 3,58 | 3,59 | 0,01 |

This stream, i.e. DMS rich stream gas phase, (see "DMS" in Fig.5) can be purified by further separation in a condenser (see "Cooling" in Fig.5), i.e. treatment step of the process. Further, methyl mercaptan, with a boiling point of 6°C, is going out from the top of said condenser, see "Cooling" and "Methyl mercaptan" in Fig.5. (The condensed stream may then also be further purified by another stripper column and another condenser.) After being purified by the further separation in the condenser (see "Cooling" in Fig.5), the purified DMS may be oxidized with nitrogen dioxide in nitrogen gas (see "NO₂/N₂" in Fig.5) to DMSO (see "DMSO" in Fig.5), and the DMSO may be further purified (see "purification" in Fig.5). During the oxidation nitrogen oxide is formed which reacted back to NO₂ with addition of ammonia. Thus, when the compound of formula (**I**) is DMSO, said treatment step comprises an oxidation step wherein DMS has been oxidized.

### Example 4:

From an acetone column total condenser (for example, "Distillation" with "Acetone+impurities"-stream in figure 1 of SE 539 579 C2), an acetone rich stream was characterized to contain see table 2, where (%) is weight %. Here in Example 4, a process is described wherein the raw material composition may be such an acetone rich stream.

**Table 2**

| Sample | MeOH (%) | EtOH (%) | Acetone (%) |
|---|---|---|---|
| 2 | 70 | 0 | 30 |

Thus, in Example 4 refer to "Crude methanol", i.e. a raw material composition which is going in to the "Biorefinery" from the left, see "Biorefinery" in Fig.2. The "Biorefinery" in Fig.2 is comprised in the process. Moreover, reduced sulphur gases may be separated, see "Reduced sulphur gases" in Fig.2, from the acetone rich stream. The acetone rich stream can be purified by further separation in a condenser (see "Pure acetone" in Fig.2). (The condensed stream may be further purified by another stripper column and another condenser.) The biorefinery of Example 4 ("Biorefinery" in Fig.2) may further comprise production of DMS, see "DMS" in Fig.2, DMSO/DMSO₂, see "DMSO/DMSO₂" in Fig.2, involving a treatment step, e.g. comprising an oxidation step see "Treatment" in Fig.2. Furthermore, the biorefinery of Example 4 ("Biorefinery" in Fig.2), may also further comprise production of acetone, see "Pure acetone" in Fig.2.

### Example 5:

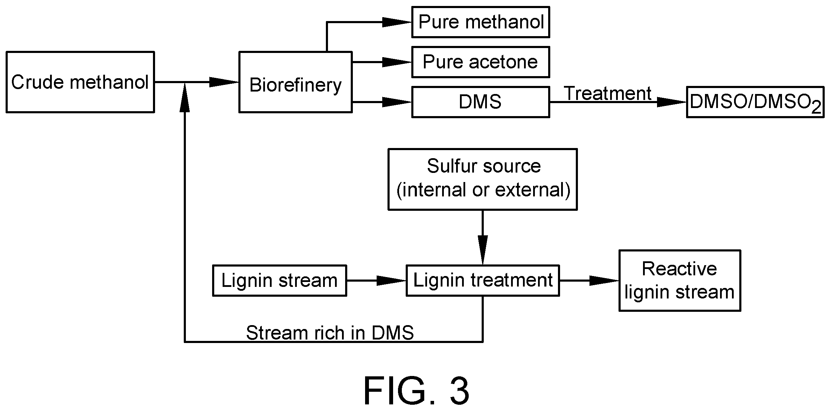

Example 5 describes a process involving production of DMS (see "DMS" in Fig. 3) and a reactive lignin product (see "Reactive lignin stream" in Fig. 3), comprising an added stream of DMS (see "Stream rich in DMS" in Fig. 3). The Example 5 (see Fig.3) comprises all parts of Example 4 (except notation that reduced sulphur gases may be separated see "Reduced sulphur gases" in Fig.2), and Example 5 further also comprises lignin treatment (see "Lignin treatment" in Fig.3) rendering said reactive lignin product and producing said added stream of DMS. The lignin treatment comprises stream of lignin (see "Lignin stream" in Fig.3) addition of internal, or external, sulphur source (see "Sulphur source (internal or external)" in Fig.3).

The lignin treatment (see "Lignin treatment" in Fig.3), comprising e.g. an alkaline treated black liquor, rendering said reactive lignin product, here a lignin with more reactive phenolic groups.

### Example 6:

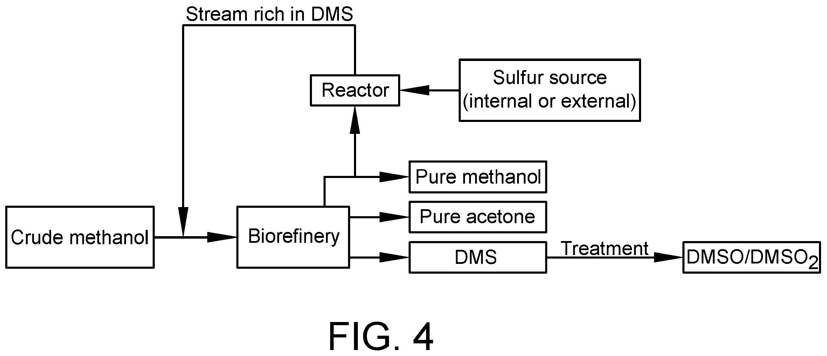

Production of DMS comprising an internal, or external, source of sulphur. Example 6 describes a process for production of DMS (see "DMS" in Fig. 4) with a recycling of a stream of DMS (see "Stream rich in DMS" in Fig. 4) to the biorefinery (see "Biorefinery" in Fig. 4).

The Example 6 (see Fig.4) comprises all parts of Example 4 (except notation that reduced sulphur gases may be separated see "Reduced sulphur gases" in Fig.2), and Example 6 may further also comprise addition of internal, or external, sulphur source (see "Sulphur source (internal or external)" in Fig.4) for reaction (see "Reactor" in Fig.4) with pure methanol (see "Pure methanol" in Fig.4), as well as, said recycling of a stream of DMS (see "Stream rich in DMS" in Fig. 4) to the raw material composition (see "Crude methanol" in Fig. 4) and to said biorefinery (see "Biorefinery" in Fig. 4).

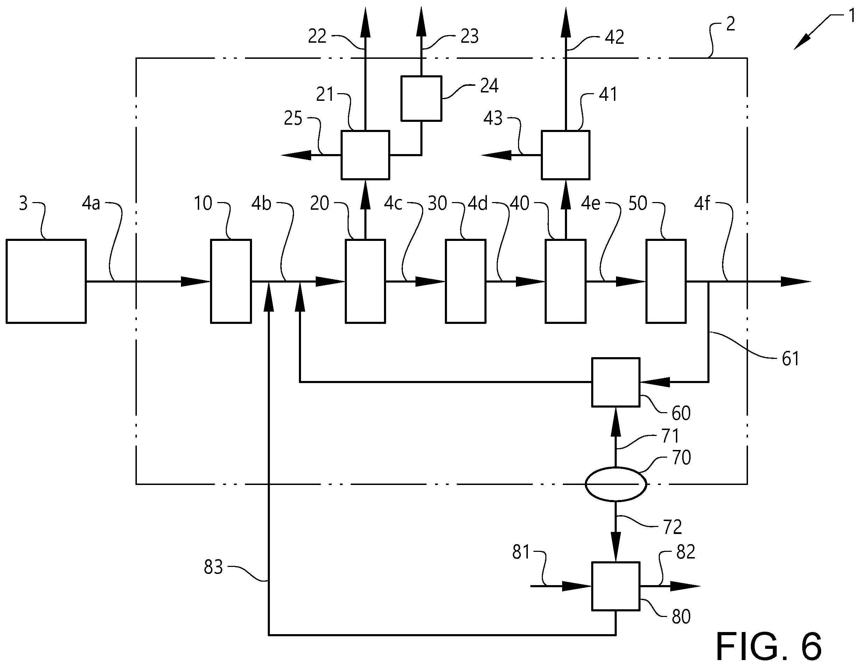

Figure 6 shows, in a schematic view, an example of a method and process plant 1 according to this disclosure. As can be seen in figure 6 the process plant 1 is configured for treatment of a stream of mixed compounds 4a obtained from a process 3 comprising decomposition and/or conversion of a wood or pulp material. Although not shown in figure 6, the process 3 may be configured for black liquor digestion of wood material and the stream of mixed compounds 4a may be a condensate from evaporation of such black liquor. The process 3 may form part of the process plant 1 and the process plant 1 may be an integrated plant comprising both a "biorefinery" (see below) and a pulp mill.

The process plant 1 comprises a processing arrangement 2, i.e. an arrangement that may be denoted "biorefinery". Figure 6 indicates that the processing arrangement 2 may comprises five treatment units 10, 20, 30, 40, 50 configured to, when the stream 4a, 4b, 4c, 4d, 4e is fed through the processing arrangement 2, separate methanol from other compounds in the stream and form a first product flow 4f containing methanol. The treatment units 10-50 may be of different type and number. In this particular example the treatment units include a first and a second primary separation unit 20, 40. The other treatment units 10, 30 and 50 are in this example optional but are indicated as it in many cases would be suitable to include further treatment units, such as further separation units that may form further product flows and/or units for acidic treatment of the flow or similar. For the principle of this disclosure only one of the first and second primary separation units 20, 40 is required.

The stream of mixed compounds is denoted 4a when fed to the processing arrangement 2. As the stream passes the treatment units 10-50 the composition and mass flow rate of the stream typically changes since some compounds is removed, such as more volatile compounds, and some compounds may be added, such as acid. This change of the stream is indicated by the notations 4b-4f used after the treatment units 10-50. As further described below, one or more compounds including "methanol" will generally remain in the stream 4a-4f and form an outgoing first product flow 4f. This first product flow 4f may be a flow of a (to some degree) purified first product or may be a mix of compounds that might be used for combustion and thus for heating purposes (possibly after having removed water in an additional treatment unit, if not already removed in e.g. treatment unit 50). In the example of figure 6 methanol is methanol, and product flows other than the first product flow relate to more volatile compounds that are separated from (or produced from) methanol present in the "main" flow 4a-4f.

The first primary separation unit 20 is arranged to separate one or more compounds other than methanol from the stream. In this example the first primary separation unit 20 is a distillation unit that separates compounds from the stream that have a lower boiling point than methanol.

A first auxiliary separation unit 21 is arranged in association with the first primary separation unit 20 so as to receive the compounds separated from methanol in the first primary separation unit 20. The first auxiliary separation unit 21 is configured to, based on deviating boiling points, separate a second compound from at least one of the other compounds separated from methanol in the first primary separation unit 20. The first auxiliary separation unit 21 may thus be a cooler/condenser. As a result of the treatment in the first auxiliary separation unit 21, the purity of the second compound increases and form a second product flow 22, 23 in the form of a purified second compound and/or a purified derivative of the second compound. See further explanation below regarding different variants for the second product flow(s).

As mentioned above, the treatment units further include the second primary separation unit 40, that is located downstream of the first separation unit 20 (in relation to the stream 4a-4f) and that also is a distillation unit arranged to separate at least methanol from other compounds in the stream based on deviating boiling points.

The processing arrangement 2 further comprises a second auxiliary separation unit 41 arranged in association with the second primary separation unit 40 so as to receive the compounds separated from methanol in the second primary separation unit 40. The second auxiliary separation unit 41 is configured to separate, again based on deviating boiling points, a third compound from at least one of the other compounds separated from methanol in the second primary separation unit 40. As a result, the purity of the third compound increases and it forms a third product flow 42 in the form of a purified third compound and/or a purified derivative of the third compound.

The processing arrangement 2 is further arranged to be capable of purifying methanol until it forms the first product flow 4f in the form of a purified methanol. As explained on various places in this disclosure, it may be beneficial to also produce a purified methanol, but it is not necessary that a purified methanol is produced.

In the example of figure 6, product flows 22, 23, 42 and 4f are intended to indicate outflow of products that are used in some way outside of the processing arrangement 2. These products, or a portion of the product flow, may alternatively be used for various purposes within the processing arrangement 2.

Further, the second compound is exemplified as dimethyl sulphide (DMS, CH3-S-CH3) or methyl mercaptan (CH3-S-H), while the third compound is exemplified as acetone (CH3-CO-CH3). Depending on the composition of the incoming stream 4a and the particular design of the processing arrangement 2 and the process plant 1, other compounds may be regarded as forming the first, second and third compounds.

Depending on the particular design of the processing arrangement 2, flows 22, 23 and 25 can represent different compounds, and at least one of these flows is optional, as is also an oxidizer 24 arranged downstream the first auxiliary separation unit 21. In one example, flow 22 represents purified methyl mercaptan, flow 23 represents purified DMS (optionally oxidized to DMSO in oxidizer 24), and flow 25 represents some other compounds (such as H₂S) separated from the stream 4b in the first primary separation unit 20. In another example flow 22 represents purified DMS, flow 23 is optional or represents purified DMSO (i.e. a portion of the DMS is fed to oxidizer 24) and flow 25 represents some other compounds, including e.g. methyl mercaptan and H₂S, separated from the stream 4b in the first primary separation unit 20. The flow 25 may serve as a source of sulphur and be fed to a "sulphur source" schematically indicated in figure 6 with reference number 70.

Besides product flows of DMS, DMSO, methyl mercaptan and sulphur compounds, including variants 22 and 23 of the second product flow, the method/process plant 1 of figure 6 is capable of generating product flows of purified methanol (first product flow 4f) and of purified acetone (third product flow 42).

As an example, the second primary separation unit 40 in the form of a distillation unit may be operated at around 61-62°C (if atmospheric pressure) to evaporate and separate acetone (boiling point 56°C) from e.g. methanol (boiling point 64.7°C) and ethanol (boiling point 78.4°C). Also water will then remain in the liquid phase. Remaining volatile compounds in the stream 4d, such as DMS (boiling point 37°C), methyl mercaptan (boiling point 6.0°C) and H₂S (boiling point -60.3°C) follow the acetone, as also some of the methanol. Flow 43 in figure 6 can be seen as representing volatile sulphur compounds that can be fed to the sulphur source 70. Flow 43 can alternatively be seen as representing methanol or a mix of compounds separated from acetone. Also the methanol may be separated from the acetone in the second auxiliary separation unit 41 (or in a not shown additional auxiliary separation unit) so as to purify acetone in the third product flow 42.

The processing arrangement 2 is further configured to be operated so as to increase a mass flow rate of the second and/or third product flow while decreasing a mass flow rate of the first product flow. This can, for instance, be done by adjusting temperature/pressure of the first and/or second primary separation units 20, 40 so that a larger fraction of the incoming stream 4b, 4d is separated and fed to the corresponding auxiliary separation unit 21, 41. By operating the auxiliary separation units 21, 41 properly, and possibly by adding further separation units, this provides for the possibility to generate a larger mass flow rate of purified DMS (and/or DMSO/methyl mercaptan) and purified acetone, while the mass flow rate of purified methanol decreases (since some methanol will be removed from the main stream 4). The method may focus completely on the output of purified second and/or third compound and no purified methanol needs to be produced.

As shown in figure 6, the exemplified process plant 1 further comprises a recirculation flow line 61 arranged to recirculate a portion of the stream 4c-4f from a point in the processing arrangement 2 downstream of the first primary separation unit 20, where DMS and/or methyl mercaptan is separated from the stream to a point in the processing arrangement 2 upstream of the first primary separation unit 20. In this case the recirculation flow line 61 starts downstream the last treatment unit 50 where the methanol may be purified to a relatively high degree, but it could instead start further upstream, even directly downstream the first primary separation unit 20 (at stream 4c).

As seen in figure 6 it is further included a DMS/methyl mercaptan production reactor 60 through which the portion of the stream 4c-4f is fed during recirculation. Further, a supply line 71 is arranged for feeding one or more sulphur compounds, such as H₂S, to the DMS/methyl mercaptan production reactor 60, wherein the sulphur compounds are capable of reacting with methanol present in the recirculated flow so as to form DMS and/or methyl mercaptan.

This way additional amounts of DMS or methyl mercaptan are produced from methanol and sulphur compounds and introduced in the stream 4b upstream of the first primary separation unit 20. The sulphur compounds are preferably obtained at least partly from flow 25 and/or flow 43 as mentioned above. The additional amounts of DMS or methyl mercaptan can then be separated in the first primary separation unit 20 and then further purified as described above.

The process plant 1 of figure 6 further comprises a lignin treatment arrangement 80 arranged to react at least a portion of a flow of incoming lignin 81, obtained from a wood or pulp decomposition and/or conversion process, with sulphur from an incoming flow 72 of sulphur compounds so as to form DMS and/or methyl mercaptan (as well as reactive lignin 82, which may be used for other purposes not directly related to the method of this disclosure). The flow 72 of sulphur compounds may include H₂S and is preferably obtained at least partly from flow 25 (see above).

The process plant 1 further comprises a flow line 83 for feeding DMS and/or methyl mercaptan from the lignin treatment arrangement 80 to a point in the processing arrangement 2 upstream of the first primary a separation unit 20 where DMS and/or methyl mercaptan is separated from the stream. Additional DMS (or DMSO or methyl mercaptan) can thus be produced, separated and purified in line with what is described above.

Besides the many modifications and variations already described above in relation to the example shown in figure 6, it may be mentioned that the recirculation arrangement 60, 61 and the lignin treatment arrangement 80, 83 are optional features. Further, another variant of an oxidized derivative of DMS, besides DMSO, is dimethyl sulfone (DMSO₂).

## Claims

1. Method for treatment of a stream of mixed compounds (4a) obtained from a process (3) comprising decomposition and/or conversion of a wood or pulp material, the method comprising:
- feeding the stream (4a-4e) through a processing arrangement (2) comprising one or more treatment units (10, 20, 30, 40, 50) arranged to separate methanol from other compounds in the stream and form a first product flow (4f) containing methanol, wherein the one or more treatment units comprises at least a first primary separation unit (20) arranged to separate one or more compounds other than methanol from the stream; and
- feeding the compounds separated from methanol in the first primary separation unit (20) to a first auxiliary separation unit (21) so as to separate a second compound from at least one of the other compounds separated from methanol in the first primary separation unit (20) and thereby increase the purity of said second compound and form a second product flow (22, 23) in the form of a purified second compound and/or a purified derivative of the second compound,
wherein the second compound is dimethyl sulfide (DMS, CH3-S-CH3), methyl mercaptan (CH3-S-H) or acetone (CH3-CO-CH3).

2. Method according to claim 1, wherein the one or more treatment units further comprises a second primary separation unit (40) arranged to separate methanol from other compounds in the stream, wherein the method comprises:
- feeding the compounds separated from methanol in the second primary separation unit (40) to a second auxiliary separation unit (41) so as to separate a third compound from at least one of the other compounds separated from methanol in the second primary separation unit (40) and thereby increase the purity of said third compound and form a third product flow (42) in the form of a purified third compound (42) or a purified derivative of the third compound,
wherein the third compound is dimethyl sulfide (DMS, CH3-S-CH3), methyl mercaptan (CH3-S-H) or acetone (CH3-CO-CH3), and wherein the second and third compounds are different compounds.

3. Method according to claim 1 or 2, wherein the second compound is dimethyl sulfide (DMS, CH3-S-CH3) or methyl mercaptan (CH3-S-H).

4. Method according to claim 2 or 3, wherein the third compound is acetone (CH3-CO-CH3).

5. Method according to any one of claims 2-4, wherein the first primary separation unit (20) is arranged upstream of the second primary separation unit (40).

6. Method according to any of the claims 3-5, wherein the method comprises:
- recirculating a portion of the stream (4c, 4d, 4e, 4f) in a recirculation flow line (61) from a point in the processing arrangement downstream of a separation unit (20) where DMS and/or methyl mercaptan is separated from the stream, to a point in the processing arrangement upstream of the separation unit (20) where DMS and/or methyl mercaptan is separated from the stream, wherein the recirculated portion of the stream (4c, 4d, 4e, 4f) during recirculation is fed through a DMS/methyl mercaptan production reactor (60) to which is fed, via a supply line (71), one or more sulphur compounds capable of reacting with methanol or another compound in stream so as to form DMS and/or methyl mercaptan.

7. Method according to any of the above claims, wherein the second compound is DMS, and wherein the method comprises:
- feeding purified DMS from the first auxiliary separation unit (21) to an oxidizing unit (24) so as to oxidize DMS and form a purified derivative of DMS (23) in the form of dimethyl sulfoxide (DMSO).

8. Method according to any of claims 3-7, wherein the method comprises:
- feeding a flow (83) containing DMS and/or methyl mercaptan from a lignin treatment arrangement (80) to a point in the processing arrangement upstream of a separation unit (20) where DMS and/or methyl mercaptan is separated from the stream, wherein the lignin treatment arrangement (80) is arranged to react at least a portion of a flow of incoming lignin (81) obtained from a wood or pulp decomposition and/or conversion process with sulphur from an incoming flow (72) of sulphur compounds so as to form DMS and/or methyl mercaptan.

9. Use of a process plant (1) for carrying out the method of claims 1-8, the process plant comprising:
- a processing arrangement (2) comprising one or more treatment units (10, 20, 30, 40, 50) configured to, when the stream (4a, 4b, 4c, 4d, 4e) is fed through the processing arrangement, separate methanol from other compounds in the stream and form a first product flow (4f) containing methanol, wherein the one or more treatment units comprises at least a first primary separation unit (20) arranged to separate one or more compounds other than methanol from the stream,
- the processing arrangement (2) further comprising a first auxiliary separation unit (21) arranged in association with the first primary separation unit (20) so as to receive the compounds separated from methanol in the first primary separation unit (20), the first auxiliary separation unit (21) being configured to separate a second compound from at least one of the other compounds separated from methanol in the first primary separation unit (20) and thereby increase the purity of said second compound and form a second product flow (22, 23) in the form of a purified second compound and/or a purified derivative of the second compound.

10. Use of a process plant (1) according to claim 9, wherein the one or more treatment units further comprises a second primary separation unit (40) arranged to separate methanol from other compounds in the stream, and wherein the processing arrangement (2) further comprising a second auxiliary separation unit (41) arranged in association with the second primary separation unit (40) so as to receive the compounds separated from methanol in the second primary separation unit (40), the second auxiliary separation unit (41) being configured to separate a third compound from at least one of the other compounds separated from methanol in the second primary separation unit (40) and thereby increase the purity of said third compound and form a third product flow (42) in the form of a purified third compound and/or a purified derivative of the third compound.

11. Use of a process plant (1) according to any of claims 9-10, wherein the processing arrangement (2) comprises at least two treatment units (10, 20, 30, 40, 50) arranged in series in relation to the flow of the stream containing methanol.

12. Use of a process plant (1) according to any one of claims 10-11, wherein the first primary separation unit (20) is arranged upstream of the second primary separation (40) unit.

13. Use of a process plant (1) according to any of claims 9-12, wherein the processing arrangement (2) comprises:
- a recirculation flow line (61) arranged to recirculate a portion of the stream (4c, 4d, 4e, 4f) from a point in the processing arrangement (2) downstream of a primary separation unit (20) where DMS and/or methyl mercaptan is separated from the stream, to a point in the processing arrangement upstream of the primary separation unit (20) where DMS and/or methyl mercaptan is separated from the stream;
- a DMS/methyl mercaptan production reactor (60) through which the portion of the stream (4c, 4d, 4e, 4f) is fed during recirculation, and
- a supply line (71) for feeding one or more sulphur compounds to the DMS/methyl mercaptan production reactor (60), wherein the sulphur compounds are capable of reacting with methanol or another compound in stream so as to form DMS and/or methyl mercaptan.

14. Use of a process plant (1) according to any of claims 9-13, wherein the processing arrangement (2) comprises an oxidizing unit (24) arranged downstream the first auxiliary separation unit (21) so as to allow oxidation of the second compound and form a purified derivative of the second compound.

15. Use of a process plant (1) according to any of claims 9-14, wherein the process plant (1) comprises a lignin treatment arrangement (80) arranged to react at least a portion of a flow of incoming lignin (81) obtained from a wood or pulp decomposition and/or conversion process with sulphur from an incoming flow (72) of sulphur compounds so as to form DMS and/or methyl mercaptan,
wherein the process plant (1) further comprises a flow line (83) for feeding DMS and/or methyl mercaptan from the lignin treatment arrangement (80) to a point in the processing arrangement upstream of a separation unit (20) where DMS and/or methyl mercaptan is separated from the stream.

## Patentansprüche

1. Verfahren zur Behandlung eines Stroms von gemischten Verbindungen (4a), der aus einem Verfahren (3) umfassend Zersetzung und/oder Umwandlung eines Holz- oder Zellstoffmaterials erhalten wird, wobei das Verfahren umfasst:
- Führen des Stroms (4a-4e) durch eine Verarbeitungsanordnung (2), die eine oder mehrere Behandlungseinheiten (10, 20, 30, 40, 50) umfasst, die dafür eingerichtet sind, Methanol von anderen Verbindungen in dem Strom zu trennen und einen ersten Produktstrom (4f) zu bilden, der Methanol enthält, wobei die eine oder mehreren Behandlungseinheiten wenigstens eine erste primäre Trenneinheit (20) umfassen, die dafür eingerichtet ist, eine oder mehrere andere Verbindungen als Methanol aus dem Strom abzutrennen; und
- Zuführen der in der ersten primären Trenneinheit (20) von Methanol getrennten Verbindungen zu einer ersten Hilfstrenneinheit (21), um eine zweite Verbindung von wenigstens einer der anderen in der ersten primären Trenneinheit (20) von Methanol getrennten Verbindungen zu trennen und dadurch die Reinheit der zweiten Verbindung zu erhöhen und einen zweiten Produktstrom (22, 23) in der Form einer gereinigten zweiten Verbindung und/oder eines gereinigten Derivats der zweiten Verbindung zu bilden,
wobei die zweite Verbindung Dimethylsulfid (DMS, CH3-S-CH3), Methylmercaptan (CH3-S-H) oder Aceton (CH3-CO-CH3) ist.

2. Verfahren nach Anspruch 1, wobei die eine oder mehreren Behandlungseinheiten ferner eine zweite primäre Trenneinheit (40) umfassen, die dafür eingerichtet ist, Methanol von anderen Verbindungen in dem Strom zu trennen, wobei das Verfahren umfasst:
- Zuführen der in der zweiten primären Trenneinheit (40) von Methanol getrennten Verbindungen zu einer zweiten Hilfstrenneinheit (41), um eine dritte Verbindung von wenigstens einer der anderen in der zweiten primären Trenneinheit (40) von Methanol getrennten Verbindungen zu trennen und dadurch die Reinheit der dritten Verbindung zu erhöhen und einen dritten Produktstrom (42) in der Form einer gereinigten dritten Verbindung (42) oder eines gereinigten Derivats der dritten Verbindung zu bilden,
wobei die dritte Verbindung Dimethylsulfid (DMS, CH3-S-CH3), Methylmercaptan (CH3-S-H) oder Aceton (CH3-CO-CH3) ist und wobei die zweite und die dritte Verbindung unterschiedliche Verbindungen sind.

3. Verfahren nach Anspruch 1 oder 2, wobei die zweite Verbindung Dimethylsulfid (DMS, CH3-S-CH3) oder Methylmercaptan (CH3-S-H) ist.

4. Verfahren nach Anspruch 2 oder 3, wobei die dritte Verbindung Aceton (CH3-CO-CH3) ist.

5. Verfahren nach einem der Ansprüche 2-4, wobei die erste primäre Trenneinheit (20) stromaufwärts der zweiten primären Trenneinheit (40) angeordnet ist.

6. Verfahren nach einem der Ansprüche 3-5, wobei das Verfahren umfasst:
- Rezirkulieren eines Teils des Stroms (4c, 4d, 4e, 4f) in einer Rezirkulationsströmungsleitung (61) von einem Punkt in der Verarbeitungsanordnung stromabwärts einer Trenneinheit (20), wo DMS und/oder Methylmercaptan von dem Strom getrennt werden, zu einem Punkt in der Verarbeitungsanordnung stromaufwärts der Trenneinheit (20), wo DMS und/oder Methylmercaptan von dem Strom getrennt werden, wobei der rezirkulierte Teil des Stroms (4c, 4d, 4e, 4f), während der Rezirkulation durch einen DMS/Methylmercaptan-Produktionsreaktor (60) geführt wird, dem über eine Zuleitung (71) eine oder mehrere Schwefelverbindungen zugeführt werden, die mit Methanol oder einer anderen Verbindung in dem Strom reagieren können, um DMS und/oder Methylmercaptan zu bilden.

7. Verfahren nach einem der vorstehenden Ansprüche, wobei die zweite Verbindung DMS ist und wobei das Verfahren umfasst:
- Zuführen von gereinigtem DMS aus der ersten Hilfstrenneinheit (21) zu einer Oxidationseinheit (24), um DMS zu oxidieren und ein gereinigtes Derivat von DMS (23) in der Form von Dimethylsulfoxid (DMSO) zu bilden.

8. Verfahren nach einem der Ansprüche 3-7, wobei das Verfahren umfasst:
- Zuführen eines Flusses (83), der DMS und/oder Methylmercaptan enthält, aus einer Ligninbehandlungsanordnung (80) zu einem Punkt in der Verarbeitungsanordnung stromaufwärts einer Trenneinheit (20), in der DMS und/oder Methylmercaptan von dem Strom getrennt werden, wobei die Ligninbehandlungsanordnung (80) dafür eingerichtet ist, wenigstens einen Teil eines Stroms von ankommendem Lignin (81), das aus einem Holz- oder Zellstoffzersetzungs- und/oder Umwandlungsverfahren erhalten wird, mit Schwefel aus einem ankommenden Strom (72) von Schwefelverbindungen umzusetzen, um DMS und/oder Methylmercaptan zu bilden.

9. Verwendung einer Verfahrensanlage (1) zur Durchführung des Verfahrens nach Ansprüchen 1-8, wobei die Verfahrensanlage umfasst:
- eine Verarbeitungsanordnung (2), die eine oder mehrere Behandlungseinheiten (10, 20, 30, 40, 50) umfasst, die dafür gestaltet sind, wenn der Strom (4a, 4b, 4c, 4d, 4e) durch die Verarbeitungsanordnung geführt wird, Methanol von anderen Verbindungen in dem Strom zu trennen und einen ersten Produktstrom (4f), der Methanol enthält, zu bilden, wobei die eine oder mehreren Behandlungseinheiten wenigstens eine erste primäre Trenneinheit (20) umfassen, die dafür eingerichtet ist, eine oder mehrere andere Verbindungen als Methanol aus dem Strom abzutrennen;
- wobei die Verarbeitungsanordnung (2) ferner eine erste Hilfstrenneinheit (21) umfasst, die in Verbindung mit der ersten primären Trenneinheit (20) dafür eingerichtet ist, die von Methanol getrennten Verbindungen in der ersten primären Trenneinheit (20) aufzunehmen, wobei die erste Hilfstrenneinheit (21) dafür gestaltet ist, eine zweite Verbindung von wenigstens einer der anderen in der ersten primären Trenneinheit (20) von Methanol getrennten Verbindungen zu trennen und dadurch die Reinheit der zweiten Verbindung zu erhöhen und einen zweiten Produktstrom (22, 23) in der Form einer gereinigten zweiten Verbindung und/oder eines gereinigten Derivats der zweiten Verbindung zu bilden.

10. Verwendung einer Verfahrensanlage (1) nach Anspruch 9, wobei die eine oder mehreren Behandlungseinheiten ferner eine zweite primäre Trenneinheit (40) umfassen, die dafür eingerichtet ist, Methanol von anderen Verbindungen in dem Strom zu trennen, und wobei die Verarbeitungsanordnung (2) ferner eine zweite Hilfstrenneinheit (41) umfasst, die in Verbindung mit der zweiten primären Trenneinheit (40) dafür eingerichtet ist, die von Methanol getrennten Verbindungen in der zweiten primären Trenneinheit (40) aufzunehmen, wobei die zweite Hilfstrenneinheit (41) dafür gestaltet ist, eine dritte Verbindung von wenigstens einer der anderen in der zweiten primären Trenneinheit (40) von Methanol getrennten Verbindungen zu trennen und dadurch die Reinheit der dritten Verbindung zu erhöhen und einen dritten Produktstrom (42) in der Form einer gereinigten dritten Verbindung und/oder eines gereinigten Derivats der dritten Verbindung zu bilden.

11. Verwendung einer Verfahrensanlage (1) nach einem der Ansprüche 9-10, wobei die Verarbeitungsanordnung (2) wenigstens zwei Behandlungseinheiten (10, 20, 30, 40, 50) umfasst, die bezogen auf den Strom des Methanolstroms in Reihe angeordnet sind.

12. Verwendung einer Verfahrensanlage (1) nach einem der Ansprüche 10-11, wobei die erste primäre Trenneinheit (20) stromaufwärts der zweiten primären Trenneinheit (40) angeordnet ist.

13. Verwendung einer Verfahrensanlage (1) nach einem der Ansprüche 9-12, wobei die Verarbeitungsanordnung (2) umfasst:
- eine Rezirkulationsströmungsleitung (61), die dafür eingerichtet ist, einen Teil des Stroms (4c, 4d, 4e, 4f) von einem Punkt in der Verarbeitungsanordnung (2) stromabwärts einer primären Trenneinheit (20), in der DMS und/oder Methylmercaptan von dem Strom getrennt werden, zu einem Punkt in der Verarbeitungsanordnung stromaufwärts der primären Trenneinheit (20), in der DMS und/oder Methylmercaptan von dem Strom getrennt werden, zu rezirkulieren;
- einen DMS/Methylmercaptan-Produktionsreaktor (60), durch den der Teil des Stroms (4c, 4d, 4e, 4f) während der Rezirkulation geführt wird, und
- eine Zuleitung (71) zum Zuführen einer oder mehrerer Schwefelverbindungen in den DMS/Methylmercaptan-Produktionsreaktor (60), wobei die Schwefelverbindungen fähig sind, mit Methanol oder einer anderen Verbindung in dem Strom zu reagieren, um DMS und/oder Methylmercaptan zu bilden.

14. Verwendung einer Verfahrensanlage (1) nach einem der Ansprüche 9-13, wobei die Verarbeitungsanordnung (2) eine Oxidationseinheit (24) umfasst, die stromabwärts der ersten Hilfstrenneinheit (21) angeordnet ist, um Oxidation der zweiten Verbindung zu ermöglichen und ein gereinigtes Derivat der zweiten Verbindung zu bilden.

15. Verwendung einer Verfahrensanlage (1) nach einem der Ansprüche 9-14, wobei die Verfahrensanlage (1) eine Ligninbehandlungsanordnung (80) umfasst, die dafür eingerichtet ist, wenigstens einen Teil eines Stroms von eingehendem Lignin (81), das aus einem Holz- oder Zellstoffzersetzungs- und/oder Umwandlungsverfahren erhalten wurde, mit Schwefel aus einem eingehenden Strom (72) von Schwefelverbindungen umzusetzen, um DMS und/oder Methylmercaptan zu bilden,
wobei die Verfahrensanlage (1) ferner eine Flussleitung (83) zum Zuführen von DMS und/oder Methylmercaptan von der Ligninbehandlungsanordnung (80) zu einem Punkt in der Verarbeitungsanordnung stromaufwärts einer Trenneinheit (20) umfasst, wo DMS und/oder Methylmercaptan von dem Strom getrennt werden.

## Revendications

1. Procédé de traitement d'un flux de composés mixtes (4a) issu d'un procédé (3) comprenant une décomposition et/ou conversion d'une matière de bois ou de pâte, le procédé comprenant :
- l'introduction du flux (4a-4e) à travers un agencement de traitement (2) comprenant une ou plusieurs unités de traitement (10, 20, 30, 40, 50) agencées pour séparer le méthanol d'autres composés dans le flux et former un premier flux de produit (4f) contenant le méthanol, la ou les unités de traitement comprenant au moins une première unité de séparation primaire (20) agencée pour séparer un ou plusieurs composés autres que le méthanol du flux ; et
- l'introduction des composés séparés du méthanol dans la première unité de séparation primaire (20) à une première unité de séparation auxiliaire (21) de manière à séparer un deuxième composé d'au moins un des autres composés séparés du méthanol dans la première unité de séparation primaire (20) et ainsi augmenter la pureté dudit deuxième composé et former un deuxième flux de produit (22, 23) sous forme d'un deuxième composé purifié et/ou d'un dérivé purifié du deuxième composé,
le deuxième composé étant le sulfure de diméthyle (DMS, CH3-S-CH3), le méthylmercaptan (CH3-S-H) ou l'acétone (CH3-CO-CH3).

2. Procédé selon la revendication 1, la ou les unités de traitement comprenant en outre une deuxième unité de séparation primaire (40) agencée pour séparer le méthanol d'autres composés dans le flux, le procédé comprenant :
- l'introduction des composés séparés du méthanol dans la deuxième unité de séparation primaire (40) à une deuxième unité de séparation auxiliaire (41) de manière à séparer un troisième composé d'au moins un des autres composés séparés du méthanol dans la deuxième unité de séparation primaire (40) et ainsi augmenter la pureté dudit troisième composé et former un troisième flux de produit (42) sous forme d'un troisième composé purifié (42) ou d'un dérivé purifié du troisième composé, le troisième composé étant le sulfure de diméthyle (DMS, CH3-S-CH3), le méthylmercaptan (CH3-S-H) ou l'acétone (CH3-CO-CH3), et les deuxième et troisième composés étant des composés différents.

3. Procédé selon la revendication 1 ou 2, le deuxième composé étant le sulfure de diméthyle (DMS, CH3-S-CH3) ou le méthylmercaptan (CH3-S-H).

4. Procédé selon la revendication 2 ou 3, le troisième composé étant l'acétone (CH3-CO-CH3).

5. Procédé selon l'une quelconque des revendications 2 à 4, la première unité de séparation primaire (20) étant agencée en amont de la deuxième unité de séparation primaire (40).

6. Procédé selon l'une quelconque des revendications 3 à 5, le procédé comprenant :
- la recirculation d'une partie du flux (4c, 4d, 4e, 4f) dans une ligne de flux de recirculation (61) d'un point de l'agencement de traitement en aval d'une unité de séparation (20) où le DMS et/ou le méthylmercaptan est séparé du flux, vers un point de l'agencement de traitement en amont de l'unité de séparation (20) où le DMS et/ou le méthylmercaptan est séparé du flux, la partie recirculée du flux (4c, 4d, 4e, 4f) lors de la recirculation étant introduite à travers un réacteur de production de DMS/méthylmercaptan (60) auquel est introduit, par l'intermédiaire d'une ligne d'alimentation (71), un ou plusieurs composés soufrés aptes à réagir avec du méthanol ou un autre composé dans le flux de manière à former du DMS et/ou du méthylmercaptan.

7. Procédé selon l'une quelconque des revendications ci-dessus, le deuxième composé étant le DMS, et le procédé comprenant :
- l'introduction du DMS purifié de la première unité de séparation auxiliaire (21) dans une unité d'oxydation (24) de manière à oxyder le DMS et former un dérivé purifié de DMS (23) sous forme de diméthylsulfoxyde (DMSO).

8. Procédé selon l'une quelconque des revendications 3 à 7, le procédé comprenant :
- l'introduction d'un flux (83) contenant du DMS et/ou du méthylmercaptan à partir d'un agencement de traitement de lignine (80) jusqu'à un point de l'agencement de traitement en amont d'une unité de séparation (20) où le DMS et/ou le méthylmercaptan est séparé du flux, l'agencement de traitement de lignine (80) étant agencé pour faire réagir au moins une partie d'un flux de lignine entrant (81) obtenu à partir d'un procédé de décomposition et/ou de conversion du bois ou de la pâte avec du soufre provenant d'un flux entrant (72) de composés soufrés de manière à former du DMS et/ou du méthylmercaptan.

9. Utilisation d'une installation de traitement (1) pour mettre en œuvre le procédé selon les revendications 1 à 8, l'installation de traitement comprenant :
- un agencement de traitement (2) comprenant une ou plusieurs unités de traitement (10, 20, 30, 40, 50) configurées pour, lorsque le flux (4a, 4b, 4c, 4d, 4e) est introduit à travers l'agencement de traitement, séparer le méthanol des autres composés dans le flux et former un premier flux de produit (4f) contenant du méthanol, la ou les unités de traitement comprenant au moins une première unité de séparation primaire (20) agencée pour séparer un ou plusieurs composés autres que le méthanol du flux ;
- l'agencement de traitement (2) comprenant en outre une première unité de séparation auxiliaire (21) agencée en association avec la première unité de séparation primaire (20) de manière à recevoir les composés séparés du methanol dans la première unité de séparation primaire (20), la première unité de séparation auxiliaire (21) étant configurée pour séparer un deuxième composé d'au moins un des autres composés séparés du méthanol dans la première unité de séparation primaire (20) et ainsi augmenter la pureté dudit deuxième composé et former un deuxième flux de produit (22, 23) sous la forme d'un deuxième composé purifié et/ou d'un dérivé purifié du deuxième composé.

10. Utilisation d'une installation de traitement (1) selon la revendication 9, la ou les unités de traitement comprenant en outre une deuxième unité de séparation primaire (40) agencée pour séparer le méthanol des autres composés dans le flux, et l'agencement de traitement (2) comprenant en outre une deuxième unité de séparation auxiliaire (41) agencée en association avec la deuxième unité de séparation primaire (40) de manière à recevoir les composés séparés du méthanol dans la deuxième unité de séparation primaire (40), la deuxième unité de séparation auxiliaire (41) étant configurée pour séparer un troisième composé d'au moins un des autres composés séparés du méthanol dans la deuxième unité de séparation primaire (40), et ainsi augmenter la pureté dudit troisième composé et former un troisième flux de produit (42) sous la forme d'un troisième composé purifié et/ou d'un dérivé purifié du troisième composé.

11. Utilisation d'une installation de traitement (1) selon l'une quelconque des revendications 9 et 10, l'agencement de traitement (2) comprenant au moins deux unités de traitement (10, 20, 30, 40, 50) agencées en série par rapport à l'écoulement du flux contenant du méthanol.

12. Utilisation d'une installation de traitement (1) selon l'une quelconque des revendications 10 et 11, la première unité de séparation primaire (20) étant agencée en amont de la deuxième unité de séparation primaire (40).

13. Utilisation d'une installation de traitement (1) selon l'une quelconque des revendications 9 à 12, l'agencement de traitement (2) comprenant :
- une ligne de flux de recirculation (61) agencée pour faire recirculer une partie du flux (4c, 4d, 4e, 4f) d'un point de l'agencement de traitement (2) en aval d'une unité de séparation primaire (20) où le DMS et/ou le méthylmercaptan sont séparés du flux, vers un point de l'agencement de traitement en amont de l'unité de séparation primaire (20) où le DMS et/ou le méthylmercaptan sont séparés du flux ;
- un réacteur de production de DMS/méthylmercaptan (60) à travers lequel la partie du flux (4c, 4d, 4e, 4f) est introduite lors de la recirculation, et
- une ligne d'alimentation (71) pour l'introduction d'un ou plusieurs composés soufrés au réacteur de production de DMS/méthylmercaptan (60), les composés soufrés étant aptes à réagir avec du méthanol ou un autre composé dans le flux de manière à former du DMS et/ou du méthylmercaptan.

14. Utilisation d'une installation de traitement (1) selon l'une quelconque des revendications 9 à 13, l'agencement de traitement (2) comprenant une unité d'oxydation (24) agencée en aval de la première unité de séparation auxiliaire (21) de manière à permettre l'oxydation du deuxième composé et former un dérivé purifié du deuxième composé.

15. Utilisation d'une installation de traitement (1) selon l'une quelconque des revendications 9 à 14, l'installation de traitement (1) comprenant un agencement de traitement de lignine (80) agencé pour faire réagir au moins une partie d'un flux de lignine entrant (81) obtenu à partir d'un procédé de décomposition et/ou de conversion de bois ou de pâte avec du soufre provenant d'un flux entrant (72) de composés soufrés de façon à former du DMS et/ou du méthylmercaptan,
l'installation de traitement (1) comprenant en outre une ligne d'écoulement (83) pour l'introduction de DMS et/ou de méthylmercaptan à partir de l'agencement de traitement de lignine (80) vers un point de l'agencement de traitement en amont d'une unité de séparation (20) où le DMS et/ou le méthylmercaptan sont séparés du flux.
